# EUROPEAN PATENT SPECIFICATION

(11) **EP 3 426 339 B1**
(45) Date of publication and mention of the grant of the patent: **10.05.2023**
(21) Application number: 17764100.8
(22) Date of filing: 09.03.2017
(51) Int. Cl.: A61N 1/36, A61N 1/05, H04R 25/00

(54) **COCHLEAR STIMULATION SYSTEM WITH SURROUND SOUND AND NOISE CANCELLATION**
COCHLEA-STIMULATIONSSYSTEM MIT RAUMKLANG- UND RAUSCHUNTERDRÜCKUNG
SYSTÈME DE STIMULATION COCHLÉAIRE DOTÉ DE SUPPRESSION DU SON ET DU BRUIT AMBIANTS

(30) Priority: 11.03.2016 US 201662306836 P
(43) Date of publication of application: 16.01.2019
(73) Proprietor: Mayo Foundation for Medical Education and Research, Rochester, MN 55905 (US)
(72) Inventor: CEVETTE, Michael J., Cave Creek, AZ 85331 (US); STEPANEK, Jan, Scottsdale Arizona 85258 (US); PRADHAN, Gaurav N., Fountain Hills Arizona 85268-2013 (US); BROOKLER, Kenneth H., Norwalk Connecticut 06855-2521 (US)
(74) Representative: Vossius & Partner Patentanwälte Rechtsanwälte mbB
(86) International application number: PCT/US2017/021579
(87) International publication number: WO 2017/156276

(56) References cited:
- EP-A1- 0 830 802
- EP-A1- 2 806 661
- DE-A1- 10 040 660
- US-A- 3 766 331
- US-A1- 2006 045 294
- US-A1- 2009 046 874
- US-A1- 2011 188 662
- US-A1- 2014 128 940
- US-A1- 2015 133 716
- US-A1- 2015 208 956
- US-A1- 2015 306 388
- US-A1- 2015 367 132

## Description

### FIELD OF THE INVENTION

The invention relates generally cochlear stimulation devices that electrically stimulate the sensation of hearing, for example in persons having hearing disabilities.

### BACKGROUND

Cochlear stimulation systems and methods for externally electrically stimulating the sensations of hearing are generally known and disclosed, for example, in the Zink U. S. Patent 3,766,331, Litvak U.S. Patent 8,126,565, Schleich U.S. Patent 8,417,348, Schleich U.S. Patent 8,948,877 and Goodman U.S. Patent 9,071,896. These systems and methods use electrodes positioned on the user's head to transmit electrical signals that stimulate the cochlea and auditory nerve to provide the sensation of hearing. There remains, however, a continuing need for improved electrically stimulated sound systems.

US2014/0128940 A1 discloses an audio-cochlear implant processor device for a hearing impaired listener.

### SUMMARY

The invention for which protection is sought is defined in independent claim 1. The dependent claims concern particular embodiments.

### BRIEF DESCRIPTION OF THE DRAWINGS

FIG. 1 is a diagrammatic illustration of a cochlear stimulation system in accordance with embodiments of the invention.
FIG. 2 is a block diagram of embodiments of the processor/control shown in FIG. 1.
FIG. 3 is diagrammatic illustration of the use of the system to stimulate sound in different directions provide perceptions of lateralization.

### DESCRIPTION OF THE INVENTION

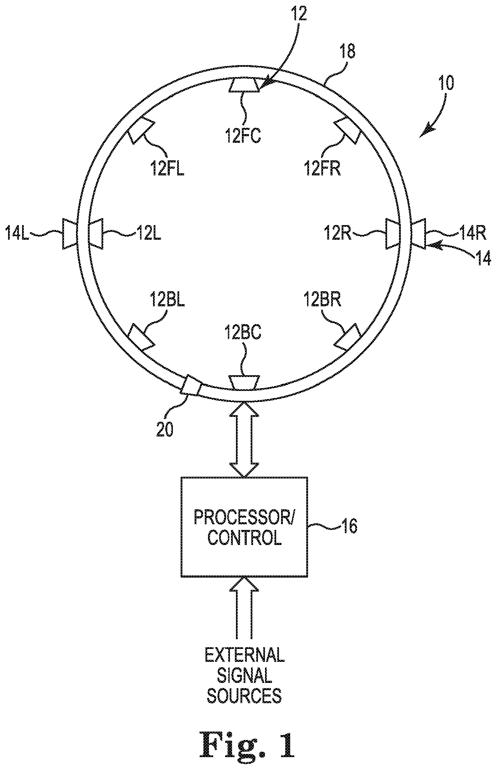

FIG. 1 illustrates a cochlear and auditory nerve stimulation system 10 having surround sound and/or noise cancellation capabilities in accordance with embodiments of the invention. As shown, system 10 includes multiple channel stimulation electrodes 12, multiple channel microphones 14, and a processor/control 16. The illustrated embodiment includes front center, front left, front right, left, right, back center, back left and back right channel electrodes 12FC, 12FL, 12FR, 12L, 12R, 12BC, 12BL and 12 BR, respectively. Other embodiments (not shown) have more or fewer electrodes 12. Right and left channel microphones 14L and 14R, respectively, are shown in FIG. 1, although other embodiments (not shown) have more or fewer microphones 14. Electrodes 12 and microphones 14 are configured to be attached to or worn at spaced apart locations around the user's head or neck. For example, in the illustrated embodiment, the electrodes 12 and microphones 14 are attached at spaced-apart locations to a structure such as a strap 18 that can be attached to the user's head or neck. A coupler, such as buckle 20 on the strap 18, can facilitate mounting the strap to the user.

Processor/control 16 has an input that can be coupled to a source of external audio signals (not shown). System 10 can, for example, be configured to interface to mobile devices, DVD players and other sources of audio content such as cable, satellite and television broadcasts and theaters. In response to the audio signals, processor/control 16 generates multiple channel surround sound electrode drive signals that are coupled to associated channel stimulation electrodes 12. The application of the surround sound drive signals to the user causes the sensation of hearing with the enriched reproduction and spatial qualities of surround sound (e.g., sequenced to have directionality and laterality). Based on timing and intensity cues, sound can be moved around the user's head. In embodiments, processor/control 16 also has an input coupled to a source, such as microphones 14, of ambient noise signals. In response to the ambient noise signals, the processor/control 16 causes the surround sound drive signals to be produced in a manner that causes global and/or directional cancellation of the ambient noise, thereby enabling enhanced hearing of the desired surround sound audio.

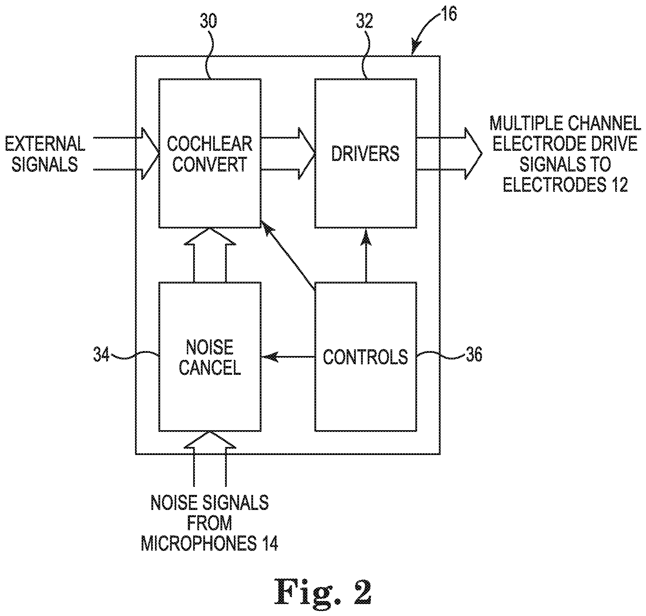

FIG. 2 is a block diagram of a processor/control 16 in accordance with embodiments of the invention. The illustrated embodiment includes a cochlear converter 30, drivers 32, noise canceler 34 and controls 36. In embodiments, components of processor/control 16 such as cochlear converter 30 and/or noise canceler 34 can be implemented, for example, as a programmed processor (with associated memory), digital signal processor and/or discrete circuit components (not illustrated). Cochlear converter 30 receives, as an input, multiple channel surround sound-formatted audio signals corresponding to each of the multiple channel electrodes 12 (e.g., front center, front left, front right, right, left, back, back left, and back right signals in the illustrated embodiment). Any of a range of known surround sound technologies and formats can be used in system 10. The converter 30 converts those inputted audio signals to corresponding multiple channel cochlear and nerve simulation signals having characteristics or signal parameters that can produce, in the user, the sensation of hearing with the audio content of the inputted audio signals. By way of example, cochlear converter 30 can use structures and/or methods of the types disclosed in the U.S. patents identified in the Background section above to provide this conversion functionality, or other known or otherwise conventional structures and/or methods. Briefly, and by way of example, cochlear converter 30 can produce low RF frequency (e.g., about 60 kHz) signals that are modulated by audio frequencies corresponding to the audio content of the associated channels.

In some embodiments, processor/control 16 receives multiple channel audio signals from the signal source, and cochlear converter 30 coverts each of those input signals to the associated stimulation signals. In other embodiments the processor/control 16 receives a single channel audio signal, or a signal having fewer channels than electrodes 12. Such embodiments can include a surround sound generator or synthesizer (not shown) that generates signals corresponding to each channel of the system 10 before those individual channel signals are processed by the cochlear converter 30.

In embodiments the channel stimulation signals produced by the cochlear converter 30 are applied to drivers 32 before being applied to the electrodes 12. Drivers 32 can, for example, include transformers or other components that convert or change the voltage and/or current levels of the stimulation signals to levels capable of providing the efficacious hearing results when the stimulation signals are applied to the user.

Noise canceler 34 receives signals representative of ambient noise in the vicinity of the user. In response to the ambient noise signals, the noise canceler 34 produces signals that, after being coupled to cochlear converter 30, will cause the cochlear converter to produce the stimulation signals in such a manner as to effectively cancel out the ambient noise. Known or otherwise conventional noise cancellation methodologies can be used for this purpose. For example, the cochlear converter 30 can produce stimulation signals having components with the same frequency and content as the ambient noise signals, but 180° out of phase with the ambient noise signals. In embodiments, the noise canceler 34 can produce control signals characteristic of the ambient noise signals. In these embodiments the cochlear converter 30 can use the control signals to control the generation of the channel stimulation signals. In other embodiments, the noise canceler 34 can produce audio signals that are summed with the audio signals applied to the cochlear converter 30 to effectively cancel the ambient noise before the audio signals are processed by the cochlear converter.

In embodiments, the noise canceler 34 functions globally by producing the same noise cancellation effects on all the channel stimulation signals. In such embodiments, for example, the ambient noise signals can be provided by either of microphones 14R and 14L. In other embodiments, the noise canceler 34 uses multiple channel ambient noise signals representative of ambient noise from different directions provided by each of several different microphones such as 14R and 14L, and causes the cochlear converter 30 to produce channel stimulation signals providing directional-specific noise cancellation. For example, the noise cancellation effects provided by the stimulation signal applied to electrodes 12FL, 12L and/or 12BL can be based upon the ambient noise signals provided by microphone 14L, and the stimulation signal applied to electrodes 12FR, 12R and/or 12BR can be based upon the ambient noise signals provided by microphone 14R. Such directional or multi-channel noise cancellation can, for example, be particularly efficacious for users having single-sided hearing loss.

Controls 36 can be coupled to cochlear converter 30, drivers 32 and/or noise canceler 34 and actuated by the user to control the system 10. By way of example, controls 36 can control the volume of the hearing provided by the stimulation signals, and the balance or relative volumes between the electrodes 12. In other embodiments, the frequency content or the tone of the hearing can be controlled by controls 36.

Other embodiments, alternatively or additionally to those described above, include an array of multiple electrodes (which can be incorporated into a contact) around the head. The linear accelerators of the inner ear, namely the otoliths, coupled with the angular accelerator of the semicircular canals have carrier frequencies ranging from approximately 10 kHz to over 60 kHz. A corresponding frequency of stimulation, which can be applied to one of the electrodes, may produce a sensation of pitch. Stimulation of the semicircular canals (e.g., by a signal applied to a second set of the electrodes), may produce a sensation of yaw and roll. Yet additional electrodes in the headband can stimulate to produce vector stimulation. Additional electrodes can have a 60 kHz carrier frequency for stimulation of hearing and binaural. Depending upon the surround sound output of a particular production, yet additional 60 kHz carrier frequency electrodes can be added (e.g., to the headband) to produce the surround sound effect.

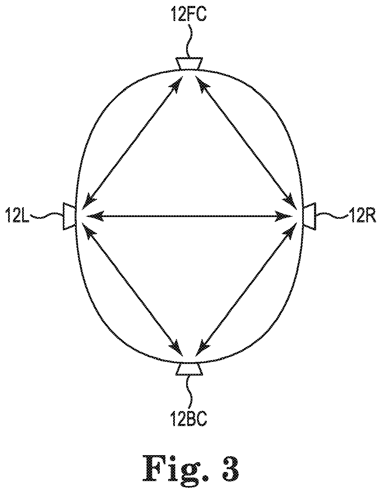

FIG. 3 is a diagrammatic illustration of front center, back center, left and right channel electrodes 12FC, 12BC, 12L and 12R, respectively, attached to a user, and lines illustrating a number of different directions of stimulation by those channel electrodes to provide the user with a perception of lateralization or different spatial locations of sound. Interaural time differences ("ITDs;" differences in time between stimulation provided by different channel electrodes) and/or interaural level differences ("ILDs;" differences in level or amplitude between stimulation provided by different channel electrodes) can be used to manipulate the perception of the location and directionality of sound (i.e., lateralization) within the head of a user. ILDs can be particularly useful to reflect the spatial locations of sounds with higher frequencies through the use of differences in sound pressure level (SPL) arriving at each ear. ITDs are particularly useful to provide the perception of lateralization at lower frequencies by timing differences for sound reaching each ear. In embodiments, ILDs and/or ITDs are provided between channel electrodes by processor/control 16 to manipulate the perception of the location of the sound (e.g., in front of, behind, left and or right of the user). By varying the ILDs and/or ITDs, the perception of dynamically varying locations of the sound can also be provided.

## Claims

1. A cochlear stimulation system (10) configured for electrically stimulating the sensation of hearing, the cochlear stimulation system comprising:
a signal input to receive an audio input signal which is a multiple channel surround sound-formatted signal; and
a multiple channel cochlear converter (30) coupled to the signal input to produce multiple channel surround sound cochlear stimulation signals based on the audio input signal;
a multiple channel microphone input (14) for receiving multiple channel ambient noise signals;.
a sound canceling processor (34) coupled between the microphone input and the multiple channel cochlear converter to produce multiple channel sound canceling signals based on the multiple channel ambient noise signals; and
wherein the cochlear converter produces the multiple channel surround sound cochlear stimulation signals further based on the multiple channel sound canceling signals, and
wherein the cochlear stimulation system further includes multiple channel cochlear stimulation electrodes (12), each of the electrodes configured for attachment to a user and coupled to the converter to receive one of the multiple channel surround sound cochlear stimulation signals.

2. The system of claim 1 wherein:
the multiple channel cochlear converter produces at least front, left and right cochlear stimulation signals; and
the multiple channel cochlear stimulation electrodes include at least front, left and right stimulation electrodes coupled to receive the front, left and right stimulation signals, respectively.

3. The system of claim 2 wherein:
the cochlear converter produces the front cochlear stimulation signals having one or more of front center, front left and front right stimulation signals; and
the front stimulation electrode includes one or more of front center, front left and front right electrodes.

4. The system of claim 3 wherein:
the cochlear converter produces the multiple channel surround sound cochlear stimulation signals having one or more of back left and back right stimulation signals; and
the multiple channel cochlear stimulation electrodes include one or more of back left and back right electrodes.

5. The system of claim 1 wherein the system further includes a surround sound signal synthesizer coupled between the signal input and the multiple channel cochlear converter, to produce the audio input signal.

6. The system of claim 1, further including a mounting device, optionally a strap (18), configured to mount the multiple channel cochlear stimulation electrodes to a user.

7. The system of claim 1 wherein the multiple channel sound canceling signals are produced by the sound canceling processor as control signals characteristic of the multiple channel ambient noise signals.

8. The system of claim 1 and further including controls, optionally including a volume control.

9. The system of claim 1 further including drivers (32) including transformers that change the voltage and/or current levels of the multiple channel cochlear stimulation signals, wherein the multiple channel surround sound cochlear stimulation signals produced by the multiple channel cochlear converter (30) are applied to the drivers before being applied to the multiple channel cochlear stimulation electrodes (12).

10. The cochlear stimulation system of claim 1 wherein the multiple channel cochlear converter produces multiple channel cochlear stimulation signals having interaural level differences.

11. The cochlear stimulation system of claim 10 wherein the multiple channel cochlear converter produces multiple channel cochlear stimulation signals having interaural time differences.

12. The cochlear stimulation system of claim 11 wherein the multiple channel cochlear converter produces multiple channel cochlear stimulation signals having changing interaural level and time differences.

13. The cochlear stimulation system of claim 1 wherein the multiple channel cochlear converter produces multiple channel cochlear stimulation signals having interaural time differences.

14. The cochlear stimulation system of claim 1
wherein the multiple channel cochlear stimulation signals include one or both of interaural level differences and interaural time differences.

## Patentansprüche

1. Cochlea-Stimulationssystem (10), das zum elektrische Stimulieren der Hörempfindung konfiguriert ist, wobei das Cochlea-Stimulationssystem aufweist:
einen Signaleingang zum Empfangen eines Audio-Eingangssignals, das ein Mehrkanal-Raumklang-formatiertes Signal ist; und
einen Mehrkanal-Cochlea-Wandler (30), der mit dem Signaleingang gekoppelt ist, um Mehrkanal-Raumklang-Cochlea-Stimulationssignale basierend auf dem Audio-Eingangssignal zu erzeugen;
einen Mehrkanal-Mikrofoneingang (14) zum Empfangen von Mehrkanal-Umgebungsgeräuschsignalen;
einen Schallunterdrückungsprozessor (34), der zwischen den Mikrofoneingang und den Mehrkanal-Cochlea-Wandler gekoppelt ist, um Mehrkanal-Schallunterdrückungssignale basierend auf den Mehrkanal-Umgebungsgeräuschsignalen zu erzeugen; und
wobei der Cochlea-Wandler die Mehrkanal-Cochlea-Raumklang-Stimulationssignale ferner basierend auf den Mehrkanal-Schallunterdrückungssignalen erzeugt, und
wobei das Cochlea-Stimulationssystem ferner Mehrkanal-Cochlea-Stimulationselektroden (12) aufweist, wobei jede der Elektroden zur Anbringung an einem Benutzer konfiguriert und mit dem Wandler gekoppelt ist, um eines der Mehrkanal-Raumklang-Cochlea-Stimulationssignale zu empfangen.

2. System nach Anspruch 1, wobei
der Mehrkanal-Cochlea-Wandler mindestens vordere, linke und rechte Cochlea-Stimulationssignale erzeugt; und
die Mehrkanal-Cochlea-Stimulationselektroden mindestens vordere, linke und rechte Stimulationselektroden aufweisen, die gekoppelt sind, die vorderen, linken bzw. rechten Stimulationssignale zu empfangen.

3. System nach Anspruch 2, wobei
der Cochlea-Wandler die vorderen Cochlea-Stimulationssignale erzeugt, die eines oder
mehrere von vorderen mittleren, vorderen linken und vorderen rechten Stimulationssignalen aufweisen; und
die vordere Stimulationselektrode eine oder mehrere Elektroden der vorderen mittleren, der vorderen linken und der vorderen rechten Elektroden enthält.

4. System nach Anspruch 3, wobei
der Cochlea-Wandler die Mehrkanal-Raumklang-Cochlea-Stimulationssignale mit einem oder mehreren Stimulationssignalen hinten links und hinten rechts erzeugt; und
die mehrkanaligen Cochlea-Stimulationselektroden eine oder mehrere hintere linke und hintere rechte Elektroden enthalten.

5. System nach Anspruch 1, wobei das System ferner einen Raumklang-Signalsynthesizer enthält, der zwischen den Signaleingang und den Mehrkanal-Cochlea-Wandler gekoppelt ist, um das Audio-Eingangssignal zu erzeugen.

6. System nach Anspruch 1, das ferner eine Befestigungsvorrichtung, optional einen Gurt (18), aufweist, die konfiguriert ist, die Mehrkanal-Cochlea-Stimulationselektroden an einem Benutzer zu befestigen.

7. System nach Anspruch 1, wobei die Mehrkanal-Schallunterdrückungssignale vom Schallunterdrückungsprozessor als Steuersignale erzeugt werden, die für die Mehrkanal-Umgebungsgeräuschsignale charakteristisch sind.

8. System nach Anspruch 1, das ferner Steuerungen aufweist, die optional eine Lautstärkeregelung aufweisen.

9. System nach Anspruch 1, das ferner Treiber (32) mit Transformatoren enthält, die die Spannungs- und/oder Strompegel der Mehrkanal-Cochlea-Stimulationssignale ändern, wobei die vom Mehrkanal-Cochlea-Wandler (30) erzeugten Mehrkanal-Raumklang-Cochlea-Stimulationssignale an die Treiber angelegt werden, bevor sie an die Mehrkanal-Cochlea-Stimulationselektroden (12) angelegt werden.

10. Cochlea-Stimulationssystem nach Anspruch 1, wobei der Mehrkanal-Cochlea-Wandler Mehrkanal-Cochlea-Stimulationssignale mit interauralen Pegeldifferenzen erzeugt.

11. Cochlea-Stimulationssystem nach Anspruch 10, wobei der Mehrkanal-Cochlea-Wandler Mehrkanal-Cochlea-Stimulationssignale mit interauralen Zeitdifferenzen erzeugt.

12. Cochlea-Stimulationssystem nach Anspruch 11, wobei der Mehrkanal-Cochlea-Wandler Mehrkanal-Cochlea-Stimulationssignale mit wechselnden interauralen Pegel- und Zeitdifferenzen erzeugt.

13. Cochlea-Stimulationssystem nach Anspruch 1, wobei der Mehrkanal-Cochlea-Wandler Mehrkanal-Cochlea-Stimulationssignale mit interauralen Zeitdifferenzen erzeugt.

14. Cochlea-Stimulationssystem nach Anspruch 1, wobei die Mehrkanal-Cochlea-Stimulationssignale interaurale Pegeldifferenzen und/oder interaurale Zeitdifferenzen enthalten.

## Revendications

1. Système de stimulation cochléaire (10) configuré pour stimuler électriquement la sensation d'audition, le système de stimulation cochléaire comprenant :
une entrée de signal destinée à recevoir un signal d'entrée audio qui est un signal formaté en son ambiant à canaux multiples ; et
un convertisseur cochléaire à canaux multiples (30) couplé à l'entrée de signal afin de produire des signaux de stimulation cochléaire à son ambiant à canaux multiples sur la base du signal d'entrée audio,
une entrée de microphone à canaux multiples (14) destinée à recevoir des signaux de bruit ambiant à canaux multiples ;
un processeur de suppression de son (34) couplé entre l'entrée de microphone et le convertisseur cochléaire à canaux multiples afin de produire des signaux de suppression de son à canaux multiples sur la base des signaux de bruit ambiant à canaux multiples ; et
dans lequel le convertisseur cochléaire produit les signaux de stimulation cochléaire à son ambiant à canaux multiples sur la base en outre des signaux de suppression de son à canaux multiples,
et
dans lequel le système de stimulation cochléaire comprend en outre des électrodes de stimulation cochléaire à canaux multiples (12), chacune des électrodes étant configurée pour être fixée sur un utilisateur et couplée au convertisseur afin de recevoir l'un des signaux de stimulation cochléaire à son ambiant à canaux multiples.

2. Système selon la revendication 1, dans lequel :
le convertisseur cochléaire à canaux multiples produit au moins des signaux de stimulation cochléaire avant, gauche et droit ; et
les électrodes de stimulation cochléaire à canaux multiples comprennent au moins des électrodes de stimulation avant, gauche et droite couplées afin de recevoir les signaux de stimulation avant, gauche et droit, respectivement.

3. Système selon la revendication 2, dans lequel :
le convertisseur cochléaire produit les signaux de stimulation cochléaire avant ayant un ou plusieurs de signaux de stimulation avant centre, avant gauche et avant droit ; et
l'électrode de stimulation avant comprend une ou plusieurs d'électrodes avant centre, avant gauche et avant droite.

4. Système selon la revendication 3, dans lequel :
le convertisseur cochléaire produit les signaux de stimulation cochléaire à son ambiant à canaux multiples ayant un ou plusieurs de signaux de stimulation arrière gauche et arrière droit ; et
les électrodes de stimulation cochléaire à canaux multiples comprennent une ou plusieurs d'électrodes arrière gauche et arrière droite.

5. Système selon la revendication 1, dans lequel le système comprend en outre un synthétiseur de signaux à son ambiant couplé entre l'entrée de signal et le convertisseur cochléaire à canaux multiples, afin de produire le signal d'entrée audio.

6. Système selon la revendication 1, comprenant en outre un dispositif d'installation, éventuellement une courroie (18), configuré pour installer les électrodes de stimulation cochléaire à canaux multiples sur un utilisateur.

7. Système selon la revendication 1, dans lequel les signaux de suppression de son à canaux multiples sont produits par le processeur de suppression de son sous forme de signaux de commande caractéristiques des signaux de bruit ambiant à canaux multiples.

8. Système selon la revendication 1 et comprenant en outre des commandes, comprenant éventuellement une commande de volume.

9. Système selon la revendication 1 comprenant en outre des pilotes (32) qui comprennent des transformateurs qui modifient les niveaux de tension et/ou de courant des signaux de stimulation cochléaire à canaux multiples, dans lequel les signaux de stimulation cochléaire à son ambiant à canaux multiples produits par le convertisseur cochléaire à canaux multiples (30) sont appliqués aux pilotes avant d'être appliqués aux électrodes de stimulation cochléaire à canaux multiples (12).

10. Système de stimulation cochléaire selon la revendication 1, dans lequel le convertisseur cochléaire à canaux multiples produit des signaux de stimulation cochléaire à canaux multiples qui présentent des différences de niveau interauriculaire.

11. Système de stimulation cochléaire selon la revendication 10, dans lequel le convertisseur cochléaire à canaux multiples produit des signaux de stimulation cochléaire à canaux multiples qui présentent des différences de durée interauriculaire.

12. Système de stimulation cochléaire selon la revendication 11, dans lequel le convertisseur cochléaire à canaux multiples produit des signaux de stimulation cochléaire à canaux multiples qui présentent des différences de niveau et de durée interauriculaire qui changent.

13. Système de stimulation cochléaire selon la revendication 1, dans lequel le convertisseur cochléaire à canaux multiples produit des signaux de stimulation cochléaire à canaux multiples qui présentent des différences de durée interauriculaire.

14. Système de stimulation cochléaire selon la revendication 1,
dans lequel les signaux de stimulation cochléaire à canaux multiples comprennent l'une ou les deux des différences de niveau interauriculaire ou des différences de durée interauriculaire.
